(19)

Europäisches
Patentamt
European
Patent Office
Office européen
des brevets

(11) **EP 4 064 229 A1**

(12) **EUROPEAN PATENT APPLICATION**

(43) Date of publication:
**28.09.2022 Bulletin 2022/39**

(21) Application number: **22154762.3**

(22) Date of filing: **02.02.2022**

(51) International Patent Classification (IPC):
**G06V 40/16** (2022.01)     **G06V 10/44** (2022.01)
**G06V 10/82** (2022.01)

(52) Cooperative Patent Classification (CPC):
**G06V 40/168; G06V 10/454; G06V 10/82;**
G06V 2201/03

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.03.2021   CN 202110300891**

(71) Applicant: **Shanghai 9th People's Hospital,
Shanghai Jiaotong
University School Of Medicine
Shanghai 200011 (CN)**

(72) Inventors:
  • **XIA, Ming**
    **Shanghai 200011 (CN)**
  • **JIANG, Hong**
    **Shanghai 200011 (CN)**

  • **LIN, Zhi Liang**
    **Shanghai 200011 (CN)**
  • **ZHENG, Yao Kun**
    **Shanghai 200011 (CN)**
  • **WANG, Jie**
    **Shanghai 200011 (CN)**
  • **ZHOU, Ren**
    **Shanghai 200011 (CN)**
  • **XU, Tian Yi**
    **Shanghai 200011 (CN)**
  • **CAO, Shuang**
    **Shanghai 200011 (CN)**

(74) Representative: **2K Patentanwälte Blasberg
Kewitz & Reichel
Partnerschaft mbB
Schumannstrasse 27
60325 Frankfurt am Main (DE)**

Remarks:
Amended claims in accordance with Rule 137(2)
EPC.

(54) **ARTIFICIAL INTELLIGENCE-BASED DIFFICULT AIRWAY EVALUATION METHOD AND DEVICE**

(57)     The disclosure relates to an artificial intelligence-based difficult airway evaluation method and device. The method includes the following steps: acquiring facial images of various postures; constructing a feature extraction network based on facial recognition, and extracting feature information of the facial images through the trained feature extraction network; and constructing a difficult airway classifier based on a machine learning algorithm, and performing difficult airway severity scoring on the extracted feature information of the facial images through the trained difficult airway classifier to obtain an evaluation result of a difficult airway. According to the present disclosure, early warning can be accurately provided for difficult airways in clinical anesthesia.

101 Acquiring facial images of various postures

102 Constructing a feature extraction network based on facial recognition, and extracting feature information of the facial images through the trained feature extraction network

103 Constructing a difficult airway classifier based on a machine learning algorithm, and performing difficult airway severity scoring on the extracted feature information of the facial images through the trained difficult airway classifier to obtain an evaluation result of a difficult airway

100

**FIG. 1**

Processed by Luminess, 75001 PARIS (FR)

EP 4 064 229 A1

**Description**

**Technical Field**

The present disclosure relates to the field of computer aided technology, and in particular to, an artificial intelligence-based difficult airway evaluation method and device.

**Background**

Tracheal intubation is an important method for anesthetists to perform airway management for patients in the general anesthesia state, and plays an important role in the aspects of maintaining unobstructed airway, ventilation and oxygen supply, and respiratory support, and keeping oxygenation, etc. However, despite the great progress and improvements in tracheal intubation technology and equipment, the incidence rate of perioperative complications and disability caused by difficult airways has not been greatly reduced, especially for unpredictable difficult airways. At present, the methods for evaluating difficult airways generally include Mallampatti grading, LEMON score, Wilson score, history of neck radiotherapy and auxiliary CT, MRI, US, etc., which are complicated in process, have not high positive evaluation values, and all have certain limitations.

**Summary**

The technical problem to be solved by the present disclosure is to provide an artificial intelligence-based difficult airway evaluation method and device, which can accurately provide early warning to the difficult airway in clinical anesthesia.
The technical solution used in the present disclosure to solve the technical problem is that an artificial intelligence-based difficult airway evaluation method is provided, including the following steps:

(1) acquiring facial images of various postures;
(2) constructing a feature extraction network based on facial recognition, and extracting feature information of the facial images through the trained feature extraction network; and
(3) constructing a difficult airway classifier based on a machine learning algorithm, and performing difficult airway severity scoring on the extracted feature information of the facial images through the trained difficult airway classifier to obtain an evaluation result of a difficult airway.

The facial images of various postures in the step (1) are posture images capable of reflecting the difficult airway, including a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image.
A deep learning feature extraction network is adopted in the step (2); the deep learning feature extraction network includes m layers of neural networks; each layer of network consists of several of a convolution layer, a pooling layer, a transposed convolution layer, and a fully connected layer; an ith layer is connected to a jth layer, $1 < i, j < m$; and the network encodes the output images to output the feature corresponding to each patient.
A loss function of a facial recognition task adopts a facial recognition loss function; the facial recognition loss function adopts an ArcFace loss function, and the ArcFace loss function is

$$L = -\frac{1}{M} \sum_{i=1}^{M} \log \frac{e^{s\left(\cos\left(\theta_{yi}+m\right)\right)}}{e^{s\left(\cos\left(\theta_{yi}+m\right)\right)} + \sum_{j=1, j \neq yi}^{n} e^{s\cos\theta_j}}$$

, wherein s is a manually set

$$W_j = \frac{W_j}{\|W_j\|}, x_i = \frac{x_i}{\|x_i\|}, \cos\theta_j = W_j^T x_i$$

parameter, $W_j$ is the weight of the $j$th layer of neural network in the deep learning feature extraction network, $x_i$ is an input feature of the ith layer of neural network in the deep learning feature extraction network, $m$ is the layer quantity of the neural networks in the deep learning feature extraction network, $M$ is the quantity of each batch of samples during training, and $n$ is the quantity of patients.

When the difficult airway classifier in the step (3) is trained, the output results of the classifier are: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age, and weight information and the airway related medical history of the patients serve as input.

The difficult airway classifier is a model based on a decision tree, and the decision tree is split according to a maximum gain principle until the termination condition is reached. The difficult airway classifier may use a plurality of decision trees for integration, and the results of the plurality of decision trees are voted to obtain the final evaluation result.

The technical solution used in the present disclosure to solve the technical problem is that an artificial intelligence-based difficult airway evaluation device is provided, including: an image information acquisition module, configured to acquire facial images of various postures; a data recording module, configured to store the facial images and difficult airway information; a feature extraction module, configured to construct a feature extraction network based on facial recognition, and extract feature information of the facial images through the trained feature extraction network; and an evaluation module, configured to construct a difficult airway classifier based on a machine learning algorithm, and perform difficult airway severity scoring on the extracted feature information of the facial images through the trained difficult airway classifier to obtain an evaluation result of a difficult airway.

The facial images of various postures acquired by the acquisition module include: a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image.

The feature extraction module adopts a deep learning feature extraction network; the deep learning feature extraction network includes m layers of neural networks; each layer of network consists of several of a convolution layer, a pooling layer, a transposed convolution layer, and a fully connected layer; an ith layer is connected to a $j$th layer, $1<i$ and $j<m$; and a loss function of a facial recognition task adopts a facial recognition loss function.

The facial recognition loss function adopts an ArcFace loss function, and the

$$L = -\frac{1}{M}\sum_{i=1}^{M}\log\frac{e^{s\left(\cos\left(\theta_{yi}+m\right)\right)}}{e^{s\left(\cos\left(\theta_{yi}+m\right)\right)}+\sum_{j=1,j\neq yi}^{n}e^{s\cos\theta_j}}$$

ArcFace loss function is $W_j = \dfrac{W_j}{\|W_j\|}, x_i = \dfrac{x_i}{\|x_i\|}, \cos\theta_j = W_j^T x_i$ , wherein s is a manually set parameter, $W_j$ is the weight of the $j$th layer of neural network in the deep learning feature extraction network, $x_i$ is an input feature of the ith layer of neural network in the deep learning feature extraction network, $m$ is the layer quantity of the neural networks in the deep learning feature extraction network, $M$ is the quantity of each batch of samples during training, and $n$ is the quantity of patients.

When the evaluation module trains the difficult airway classifier, the output results of the classifier are: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age and weight information and the airway related medical history of the patients serve as input.

Beneficial effects

Due to the adoption of the above technical solution, compared with the prior art, the present disclosure has the following advantages and positive benefits: the feature information of the patients is extracted by the feature extraction network based on facial recognition, so that manual feature selection and image marking are avoided, and the advantage of automation is achieved; the classifier constructed by the machine learning method is used to perform difficult airway severity scoring so that the overfitting phenomenon is avoided, and early warning can be accurately provided for the difficult airway in clinical anesthesia.

**Brief Description of the Drawings**

FIG. 1 is a flowchart of an embodiment of the present disclosure;

FIG. 2 is a schematic diagram of facial images of 9 different postures in an embodiment of the present disclosure;

FIG. 3 is a schematic diagram of a feature extraction network and a difficult airway classifier in an embodiment of the present disclosure;

FIG. 4 is a schematic diagram of an accuracy rate of a facial recognition task in an embodiment of the present disclosure;

FIG. 5 is an ROC curve chart of prediction of a difficult airway score model in an embodiment of the present disclosure; and

FIG. 6 is a structural schematic diagram of an embodiment of the present disclosure.

**Detailed Description of the Embodiments**

The present disclosure will be described in detail below in conjunction with specific embodiments. It should be understood that these embodiments are only used to describe the present disclosure and are not intended to limit the scope of the present disclosure. In addition, it should be understood that those skilled in the art may make various changes or modifications to the present disclosure after reading the content taught by the present disclosure, and these equivalent forms also fall within the scope defined by the appended claims of the present application.

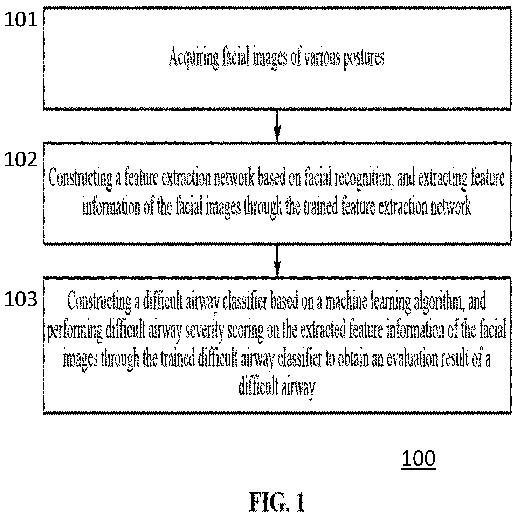

An embodiment of the present disclosure relates to an artificial intelligence-based difficult airway evaluation method. As shown in FIG. 1, the method 100 includes the following steps: 101, acquiring facial images of various postures; 102, constructing a feature extraction network based on facial recognition, and extracting feature information of the facial images through the trained feature extraction network; and 103, constructing a difficult airway classifier based on a machine learning algorithm, and performing difficult airway severity scoring on the extracted feature information of the facial images through the trained difficult airway classifier to obtain an evaluation result of a difficult airway.

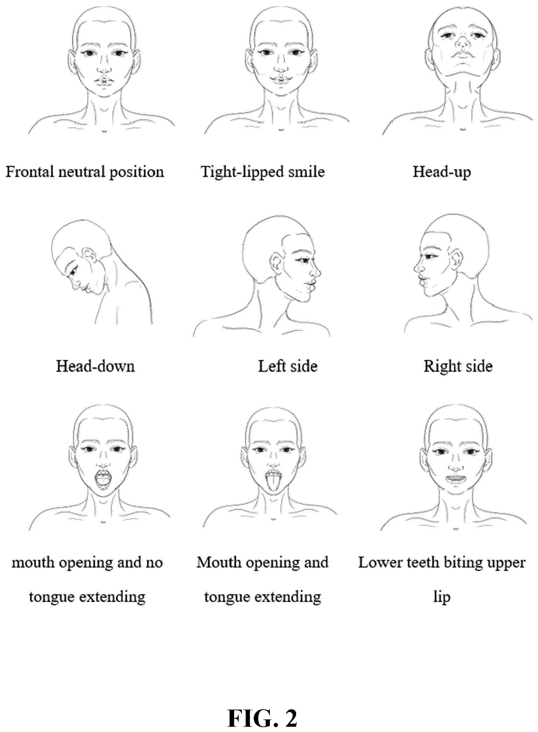

The step 101 of acquiring the facial images of various postures is specifically as follows: a photostudio is set up to collect multi-modal data of recruited patients, wherein the data includes facial images of patients with different postures on the front and side. When a patient enters the photostudio, the doctor first checks the identity and registers a series of information such as the name, gender, age, admission number, clinic department, bed number, out-patient number, height, weight, race, and operation type of the patient. Then the patient is required to do different facial movements and head movements. As shown in FIG. 2, these movements include: frontal neural position, frontal tight-lipped smile, head up, head down, neck side rotation, mouth opening and tongue extending, mouth opening without tongue extending, and lower teeth biting upper lip. The 9 different movements made by the patient are repeated for 3 times, and the optimal extreme movements of the patient are collected.

Picture data sorting:

data is named and classified, pictures of the nine different postures of the same subject are stored in the same folder which is named with a screening number, other information of the patient such as age, gender, height, weight, and various difficult airway grades, and the information such as the score results (assuming that grades I and II are 0 point, and grades III and IV are 1 point) given according to Cormack-Lehane score are stored in a database, and the serial number corresponds to the name of the folder of the pictures.

Filtration of background information of photos: the background information of the photos is filtered out by the doctor through an automatic image interception program, and only the photos of different postures of the patient for AI deep learning are reserved. The process can reduce the signal interference except the facial information during deep learning.

Data cleaning: the pictures are named, upper case, lower case and space are uniformly processed, samples with incomplete information (picture loss and grade loss) are eliminated, multiple batches of database information and pictures are merged, and the pictures are sorted to form a data set of the facial recognition task.

Data splitting of a training set and a test set and fairness verification: samples of the training set and the test set are split according to 8:2; and in order to achieve data splitting balance of the training set and the test set and the fairness verification of the test set, the feature extraction network and classifier training are performed in the training set for the patients. Then the classifier is tested by the patients, i.e., the test set, completely different from the training set; and the original data is divided into the training set and the test set at random.

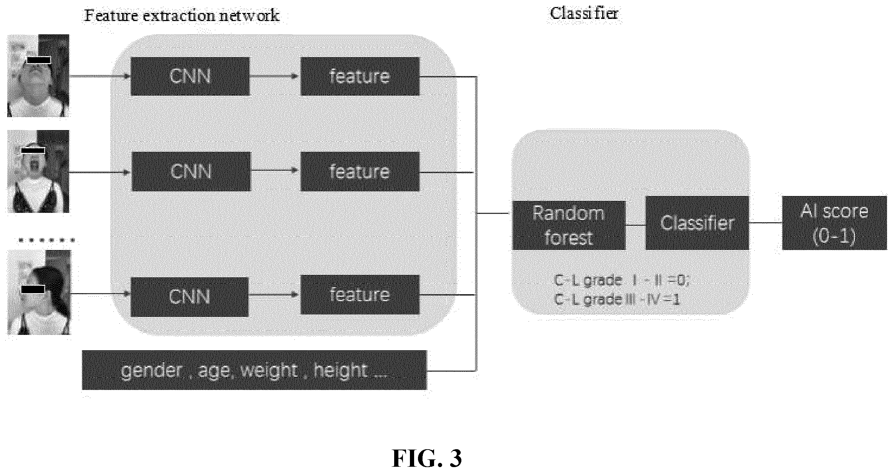

The embodiment is formed by the combination of the feature extraction network and the difficult airway classifier (as shown in FIG. 3). The information input by the model is the information such as pictures of different postures, height, weight, gender, age, and airway related medical history of the patient; and the output task is about the difficult airway severity score. The difficult airway score takes C-L grade as standard, the C-L grades I-II are defined as non-difficult airways, namely 0 point, the C-L grades III-IV are defined as difficult airways, namely 1 point, the model uses a regression analysis algorithm of the facial features and information to form a model capable of performing difficult airway severity scoring, then the feature extraction network is applied to the test set for verification, the difficult airway is scored, the closer the score is to 1 point, the higher the degree of difficulty.

The feature extraction network includes m layers of neural networks, each layer of network consists of several of a convolution layer, a pooling layer, a transposed convolution layer, and a fully connected layer, and an ith layer may be connected to a *j*th layer (1<*i,j*<*m*). The network encodes the output image to output the features corresponding to each patient, the picture of each posture of each patient outputs 4096-dimensional features, and the pictures of the total 9 postures output 36864-dimensional features.

The feature extraction network is trained according to a facial recognition loss function. The loss function is:

$$L = -\frac{1}{M}\sum_{i=1}^{m}\log\frac{e^{s(\cos(\theta_{y_i}+m))}}{e^{s(\cos(\theta_{y_i}+m))}+\sum_{j=1,j\neq y_i}^{n}e^{s\cos\theta_{y_i}}}$$

$$W_j = \frac{W_j}{\|W_j\|}, x_i = \frac{x_i}{\|x_i\|}, \cos\theta_j = W_j^T x_i$$

wherein s is a manually set parameter, $W_j$ is the weight of the *j*th layer of neural network in the deep learning feature extraction network, $x_i$ is an input feature of the ith layer of neural network in the deep learning feature extraction network, *m* is the layer quantity of the neural networks in the deep learning feature extraction network, *M* is the quantity of each batch of samples during training, and *n* is the quantity of categories, that is, the quantity of patients in the embodiment.

Data augmentation: in order to improve the accuracy of facial recognition based on deep learning, an original image is randomly cropped (a length-width ratio is maintained) and is overturned horizontally and vertically, so that image data is augmented, the performance of the facial recognition algorithm is improved, and finally, all the pictures are transformed into pictures of 112×112 for training the feature extraction network based on a facial recognition task.

The feature extraction network is trained by a stochastic gradient descent method and through weight decay and momentum modes, and the training includes 100-epoch iteration on the training set.

The difficult airway classifier adopts a random forest algorithm, and the features of each patient output by the feature extraction network, with 36864 dimensions, and totally 36868 dimensions with the addition of gender, age, height, and weight are input. A real label is the correlation between the facial features and the difficult airways of the C-L grades I-II patients is 0 point, and the correlation between the facial features and the difficult airways of the C-L grades III-IV patients is 1 point.

For the input features of the patient, the features are normalized, that is, the mean value is subtracted from each dimension of the feature and then the feature is divided by the variance.

When the difficult airway classifier is trained, the input patient samples are balanced, so that the patient samples with difficult airways and the patient samples with simple airways, which are input into the model, are the same.

For the random forest model, 600 decision trees are adopted, and cross entropy is selected to be an evaluation mode. During training, for different decision trees, the random sampling with replacement is performed in the samples for training.

A model capable of performing difficult airway severity scoring is formed by the facial features and information algorithm, and the difficult airway can be classified through a mode of setting a demarcation point according to the scoring model. The random forest algorithm has the advantages of difficulty in overfitting, capability of evaluating the importance of each feature, high anti-noise ability, etc. The basic principle is that random forest sorts the importance of information such as the facial features and the difficult airway related medical history of the patient through a plurality of decision trees, the random forest integrates the thought of learning and judging the difficult airway, the probability of the difficult airway of the patient is voted by the plurality of decision trees, and the average probability voted by all the decision trees serves as the final score result.

During the five months from August 2020 to January 2021, we recorded 4474 patients. 3931 patients have the basic fact of performing difficult airway scoring training. Table shows the baseline features of the patients used in this study.

Table 1 Baseline features of the patients in the score model

|  | Mean | [Min,Max] | Std |
|---|---|---|---|
| Age | 36 | [4,92] | 18.5 |
| Height (cm) | 164.5 | [72,194] | 11.3 |
| Weight (Kg) | 61.8 | [13,172] | 93.1 |
| Gender (M/F) | 1780/2151 |  |  |
| Total | 3931 |  |  |
|  |  |  |  |

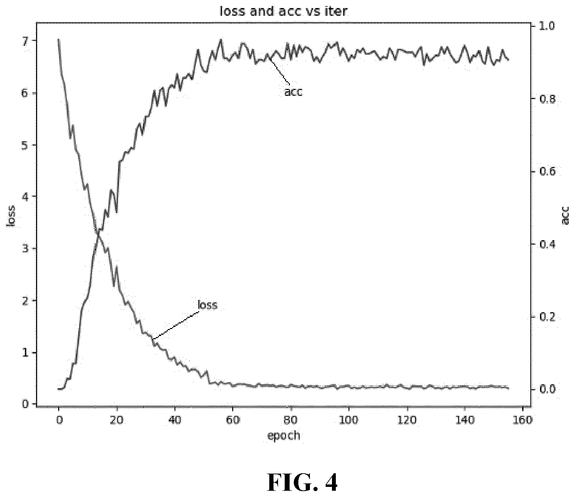

Finally, 3931 patients were included to be subjected to AI analysis. Through repeated training tests, when our facial recognition model training parameters are: learning rate: 0.003, weight decay: 0.0005 and momentum: 0.9, the best facial recognition can be achieved. The accuracy rate of the facial recognition task can reach 92% or above (as shown in FIG. 4).

The samples in the training set and the test set are randomly split according to 8:2. Through random sampling, there are totally 3144 people in the training set for performing difficult airway scoring training, wherein there are 2911 people in the non-difficult airway group (C-L grades I-II), and there are 233 people in the difficult airway group (C-L grades III-IV); and there are totally 787 people in the test set, wherein there are 729 people in the non-difficult airway group (C-L grades I-II), and there are 58 people in the difficult airway group (C-L grades III-IV) (as shown in Table 2).

Table 2 The sample quantity of different labels in the training set and the test set of the model

| Data / Label / Sample quantity | Training set (people) | Test set (people) |
|---|---|---|
| Non-difficult | 2911 | 729 |
| Difficult | 233 | 58 |
| Total | 3144 | 787 |

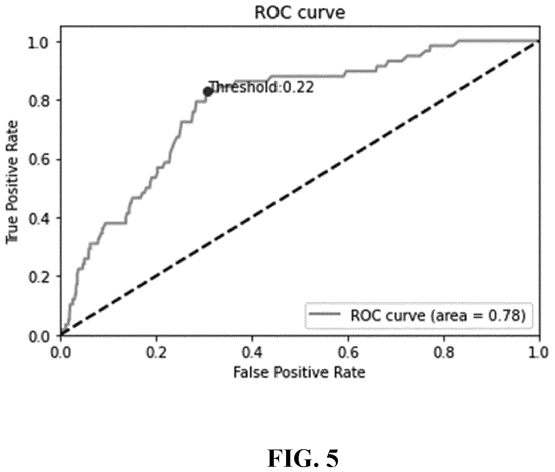

FIG. 5 is an ROC curve of the difficult airway score model for difficult airway recognition in the data set. The AUC value is 0.78. When the Youden index is an optimal boundary value for the difficult airway, the optimal boundary value of the difficult airway is 0.22 point.

In the embodiment, the boundary value for the score model to evaluate the difficult airway is determined according to the Youden index, the boundary threshold is 0.22 point (as shown in FIG. 5), the prediction result is that the difficult airway is present when the score made by the model is greater than or equal to 0.22 point, and the prediction result is that the non-difficult airway is present when the score made by the model is less than 0.22 point. The model predicts 48 true-positive patients, 10 false-negative patients, 506 true-negative patients and 233 false-positive patients in the test set including 787 patients; and the score model has the sensitivity for the difficult airway being 82.8%, the specificity being 69.4%, the positive predictive value being 18%, and the negative predictive value being 98% when predicting the difficult airway, showing high recognition performance (as shown in Table 3).

Table 3 Gold standard*model prediction result cross list

| Detection result / Gold standard result | Difficult | Non-difficult | |
|---|---|---|---|
| Difficult | 48 | 10 | Sensitivity 82.8% |
| Non-difficult | 223 | 506 | Specificity 69.4% |
| | Positive predictive value 18% | Negative predictive value 98% | |

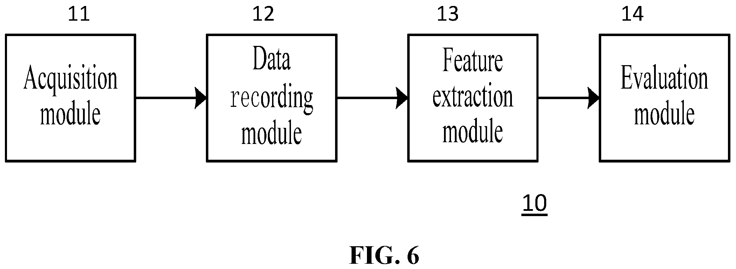

An embodiment of the present disclosure further relates to an artificial intelligence-based difficult airway evaluation device 10, as shown in FIG. 6, including: an acquisition module 11, configured to acquire facial images of various postures; a data recording module 12, configured to store the facial images and difficult airway information; a feature extraction module 13, configured to construct a feature extraction network based on facial recognition, and extract feature information of the facial images through the trained feature extraction network; and an evaluation module 14, configured to construct a difficult airway classifier based on a random forest algorithm, and perform difficult airway severity scoring on the extracted feature information of the facial images through the trained difficult airway classifier to obtain an evaluation result of a difficult airway.

The facial images of various postures acquired by the acquisition module include: a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image. An image of the specific posture of the head and neck of a patient is collected by optical imaging equipment, and the patient is located at the center of the image without interference background.

The data recording module stores the facial images, difficult airway information, height, weight, gender, and other information of the patient by utilizing a database.

The feature extraction module imports the facial images of the patient and the identity number of the patient from the data recording module, a corresponding program runs on a computer, and the feature extraction network based on facial recognition is constructed by a facial recognition loss function. The features corresponding to the facial images of the patient may be output by the feature extraction network.

The evaluation module runs the corresponding computer program, and the features extracted by the feature extraction module through the images of different postures of the patient, and the age, gender, height, and weight information of the patient are input; the real label is the difficult airway information of the patient in the data recording module, wherein the correlation between the facial features and the difficult airways of the C-L grade I-II patients is 0 point, and the correlation between the facial features and the difficult airways of the C-L grade III-IV patients is 1 point; all the features are input into the model; the classifier based on the machine learning method is trained; and the difficult airway grade of the patient may be output by the evaluation module.

It is not difficult to find that the feature information of the patient is extracted by the facial recognition-based feature, so that manual feature selection and image marking are avoided, and the advantage of automation is achieved; the classifier constructed by the machine learning method is used to perform difficult airway severity scoring so that the overfitting phenomenon is avoided, and early warning can be accurately provided for the difficult airway in clinical anesthesia.

**Claims**

1.  An artificial intelligence-based difficult airway evaluation method (100), comprising the following steps:

    (101) acquiring facial images of various postures;
    (102) constructing a feature extraction network based on facial recognition, and extracting feature information of the facial images through the trained feature extraction network; and
    (103) constructing a difficult airway classifier based on a machine learning algorithm, and performing difficult airway severity scoring on the extracted feature information of the facial images through the trained difficult airway classifier to obtain an evaluation result of a difficult airway.

2.  The artificial intelligence-based difficult airway evaluation method according to claim 1, wherein the facial images of various postures in the step (101) are posture images capable of reflecting the difficult airway, comprising a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image.

3.  The artificial intelligence-based difficult airway evaluation method according to claim 1, wherein a deep learning feature extraction network is adopted in the step (102); the deep learning feature extraction network comprises m layers of neural networks; each layer of network consists of several of a convolution layer, a pooling layer, a transposed convolution layer, and a fully connected layer; an ith layer is connected to a *j*th layer, 1<*i, j<m;* and a loss function of a facial recognition task adopts a facial recognition loss function.

4.  The artificial intelligence-based difficult airway evaluation method according to claim 3, wherein the facial recognition loss function adopts an ArcFace loss function, and the ArcFace loss

$$L = -\frac{1}{M}\sum_{i=1}^{M}\log\frac{e^{s\left(\cos\left(\theta_{yi}+m\right)\right)}}{e^{s\left(\cos\left(\theta_{yi}+m\right)\right)}+\sum_{j=1,j\neq yi}^{n}e^{s\cos\theta_j}}$$

$$W_j = \frac{W_j}{\|W_j\|}, x_i = \frac{x_i}{\|x_i\|}, \cos\theta_j = W_j^T x_i$$

    function is , wherein s is a manually set parameter, $W_j$ is the weight of the *j*th layer of neural network in the deep learning feature extraction network, $x_i$ is an input feature of the ith layer of neural network in the deep learning feature extraction network, m is the layer quantity of the neural networks in the deep learning feature extraction network, *M* is the quantity of each batch of samples during training, and *n* is the quantity of patients.

5.  The artificial intelligence-based difficult airway evaluation method according to claim 1, wherein when the difficult airway classifier in the step (103) is trained, the output result of the classifier is: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age and weight information and the airway related medical history of the patients serve as input.

6.  An artificial intelligence-based difficult airway evaluation device (10), comprising: an acquisition module (11), configured to acquire facial images of various postures; a data recording module (12), configured to store the facial images and difficult airway information; a feature extraction module (13), configured to construct a feature extraction network based on facial recognition, and extract feature information of the facial images through the trained feature extraction network; and an evaluation module (14), configured to construct a difficult airway classifier based on a machine learning algorithm, and perform difficult airway severity scoring on the extracted feature information of the facial images through the trained difficult airway classifier to obtain an evaluation result of a difficult airway.

7.  The artificial intelligence-based difficult airway evaluation device according to claim 6, wherein the facial images of various postures acquired by the acquisition module (11) comprise: a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image.

8. The artificial intelligence-based difficult airway evaluation device according to claim 6, wherein the feature extraction module (13) adopts a deep learning feature extraction network; the deep learning feature extraction network comprises m layers of neural networks; each layer of network consists of several of a convolution layer, a pooling layer, a transposed convolution layer and a fully connected layer; an ith layer is connected to a *j*th layer, 1<*i, j<m;* and a loss function of a facial recognition task adopts a facial recognition loss function.

9. The artificial intelligence-based difficult airway evaluation device according to claim 8, wherein the facial recognition loss function adopts an ArcFace loss function, and the ArcFace loss

$$L = -\frac{1}{M}\sum_{i=1}^{M}\log\frac{e^{s\left(\cos\left(\theta_{yi}+m\right)\right)}}{e^{s\left(\cos\left(\theta_{yi}+m\right)\right)}+\sum_{j=1,j\neq yi}^{n}e^{s\cos\theta_{j}}}$$

function is $W_{j}=\dfrac{W_{j}}{\left\|W_{j}\right\|}, x_{i}=\dfrac{x_{i}}{\left\|x_{i}\right\|}, \cos\theta_{j}=W_{j}^{T}x_{i}$ , wherein s is a manually set parameter, $W_{j}$ is the weight of the *j*th layer of neural network in the deep learning feature extraction network, $x_{i}$ is an input feature of the ith layer of neural network in the deep learning feature extraction network, *m* is the layer quantity of the neural networks in the deep learning feature extraction network, *M* is the quantity of each batch of samples during training, and *n* is the quantity of patients.

10. The artificial intelligence-based difficult airway evaluation device according to claim 6, wherein when the evaluation module (14) trains the difficult airway classifier, the output results of the classifier are: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age and weight information and the airway related medical history of the patients serve as input.

**Amended claims in accordance with Rule 137(2) EPC.**

1. An artificial intelligence-based difficult airway evaluation method (100), comprising the following steps:

   (101) acquiring facial images of various postures, comprising a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image;
   (102) constructing a feature extraction network based on facial recognition, and extracting feature information of the facial images through the trained feature extraction network, wherein the facial recognition loss function adopts an ArcFace loss function, and the ArcFace loss function

$$L = -\frac{1}{M}\sum_{i=1}^{M}\log\frac{e^{s\left(\cos\left(\theta_{yi}+m\right)\right)}}{e^{s\left(\cos\left(\theta_{yi}+m\right)\right)}+\sum_{j=1,j\neq yi}^{n}e^{s\cos\theta_{j}}}$$

   is

$$W_j = \frac{W_j}{\|W_j\|}, \, x_i = \frac{x_i}{\|x_i\|}, \cos\theta_j = W_j^T x_i$$

,

wherein s is a manually set parameter, $W_j$ is the weight of the $j$th layer of neural network in the deep learning feature extraction network, $x_i$ is an input feature of the $i$th layer of neural network in the deep learning feature extraction network, $m$ is the layer quantity of the neural networks in the deep learning feature extraction network, $M$ is the quantity of each batch of samples during training, and $n$ is the quantity of patients; and

(103) constructing a difficult airway classifier based on a machine learning algorithm, and performing difficult airway severity scoring on the extracted feature information of the facial images through the trained difficult airway classifier to obtain an evaluation result of a difficult airway; the information input by the difficult airway classifier is the information such as the facial images, height, weight, gender, age, and airway related medical history of the patient, the feature extraction network is applied to the test set for verification, the difficult airway is scored, the closer the score is to 1 point, the higher the degree of difficulty.

2. The artificial intelligence-based difficult airway evaluation method according to claim 1, wherein a deep learning feature extraction network is adopted in the step (102); the deep learning feature extraction network comprises $m$ layers of neural networks; each layer of network consists of several of a convolution layer, a pooling layer, a transposed convolution layer, and a fully connected layer; an $i$th layer is connected to a $j$th layer, $1 < i, j < m$; and a loss function of a facial recognition task adopts a facial recognition loss function.

3. The artificial intelligence-based difficult airway evaluation method according to claim 1, wherein when the difficult airway classifier in the step (103) is trained, the output result of the classifier is: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age and weight information and the airway related medical history of the patients serve as input.

4. An artificial intelligence-based difficult airway evaluation device (10), comprising: an acquisition module (11), configured to acquire facial images of various postures, comprising a frontal neutral position facial image, a tight-lipped smile facial image, a head-up facial image, a head-down facial image, a left-side facial image, a right-side facial image, a mouth-opening-and-no-tongue-extending facial image, a mouth-opening-and-tongue-extending facial image, and a lower-teeth-biting-upper-lip facial image; a data recording module (12), configured to store the facial images and difficult airway information; a feature extraction module (13), configured to construct a feature extraction network based on facial recognition, and extract feature information of the facial images through the trained feature extraction network, wherein the facial recognition loss function adopts an ArcFace loss function, and the ArcFace loss function is

$$L = -\frac{1}{M} \sum_{i=1}^{M} \log \frac{e^{s\left(\cos(\theta_{y_i}+m)\right)}}{e^{s\left(\cos(\theta_{y_i}+m)\right)} + \sum_{j=1, j \neq y_i}^{n} e^{s\cos\theta_j}}$$

$$W_j = \frac{W_j}{\|W_j\|}, \, x_i = \frac{x_i}{\|x_i\|}, \cos\theta_j = W_j^T x_i$$

, wherein s is a manually set parameter, $W_j$ is the weight of the $j$th layer of neural network in the deep learning feature extraction network, $x_i$ is an input feature of the $i$th layer of neural network in the deep learning feature extraction network, $m$ is the layer quantity of the neural networks in the deep learning feature extraction network, $M$ is the quantity of each batch of samples during training, and $n$ is the quantity of patients; and an evaluation module (14), configured to construct a difficult airway classifier based on a machine learning algorithm, and perform difficult airway severity scoring on the extracted feature information of the facial images through the trained difficult airway classifier to obtain an evaluation result of a difficult airway, wherein the information input by the difficult airway classifier is the information such as the facial images, height, weight, gender, age, and airway related medical history of the patient,the feature extraction network is applied to the test set for verification, the difficult airway is scored, the closer the score is to 1 point, the higher the degree of difficulty.

5.  The artificial intelligence-based difficult airway evaluation device according to claim 4, wherein the feature extraction module (13) adopts a deep learning feature extraction network; the deep learning feature extraction network comprises $m$ layers of neural networks; each layer of network consists of several of a convolution layer, a pooling layer, a transposed convolution layer and a fully connected layer; an $i$th layer is connected to a $j$th layer, $1 < i, j < m$; and a loss function of a facial recognition task adopts a facial recognition loss function.

6.  The artificial intelligence-based difficult airway evaluation device according to claim 4, wherein when the evaluation module (14) trains the difficult airway classifier, the output results of the classifier are: the correlation between facial features and difficult airways of grade I-II patients is 0 point based on Cormack-Lehane score, the correlation between facial features and difficult airways of grade III-IV patients is 1 point based on Cormack-Lehane score; and the feature information of the facial images, and the height, age and weight information and the airway related medical history of the patients serve as input.

101 Acquiring facial images of various postures

102 Constructing a feature extraction network based on facial recognition, and extracting feature information of the facial images through the trained feature extraction network

103 Constructing a difficult airway classifier based on a machine learning algorithm, and performing difficult airway severity scoring on the extracted feature information of the facial images through the trained difficult airway classifier to obtain an evaluation result of a difficult airway

100

**FIG. 1**

Frontal neutral position        Tight-lipped smile        Head-up

Head-down        Left side        Right side

mouth opening and no        Mouth opening and        Lower teeth biting upper

tongue extending        tongue extending        lip

**FIG. 2**

**FIG. 3**

FIG. 4

**FIG. 5**

FIG. 6

Europäisches
Patentamt

European
Patent Office

Office européen
des brevets

# EUROPEAN SEARCH REPORT

Application Number

EP 22 15 4762

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CUENDET GABRIEL LOUIS ET AL: "Facial Image Analysis for Fully Automatic Prediction of Difficult Endotracheal Intubation", IEEE TRANSACTIONS ON BIOMEDICAL ENGINEERING, IEEE, USA, vol. 63, no. 2, 1 February 2016 (2016-02-01), pages 328-339, XP011595457, ISSN: 0018-9294, DOI: 10.1109/TBME.2015.2457032 [retrieved on 2016-01-18] * sections I-III; page 328 - page 336 * ----- | 1-10 | INV. G06V40/16 G06V10/44 G06V10/82 |
| X | US 2010/312144 A1 (CONNOR CHRISTOPHER W [US] ET AL) 9 December 2010 (2010-12-09) * figures 1-6 * * paragraph [0023] - paragraph [0041] * ----- | 1-10 | |
| X | US 2016/278670 A1 (SCHOETTKER PATRICK [CH] ET AL) 29 September 2016 (2016-09-29) * figures 1-8 * * paragraph [0063] - paragraph [0141] * ----- | 1-10 | TECHNICAL FIELDS SEARCHED (IPC) G06V |
| X | US 2021/034841 A1 (SEGAL B SCOTT [US] ET AL) 4 February 2021 (2021-02-04) * figures 1-3 * * paragraph [0025] - paragraph [0039] * ----- | 1-10 | |

-/--

The present search report has been drawn up for all claims

| Place of search | Date of completion of the search | Examiner |
|---|---|---|
| The Hague | 4 July 2022 | Chehade, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 22 15 4762

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DENG JIANKANG ET AL: "ArcFace: Additive Angular Margin Loss for Deep Face Recognition", 2019 IEEE/CVF CONFERENCE ON COMPUTER VISION AND PATTERN RECOGNITION (CVPR), IEEE, 15 June 2019 (2019-06-15), pages 4685-4694, XP033686744, DOI: 10.1109/CVPR.2019.00482 [retrieved on 2020-01-08] * the whole document * ----- | 3,4,8,9 | |
| | | | **TECHNICAL FIELDS SEARCHED (IPC)** |

The present search report has been drawn up for all claims

| Place of search | Date of completion of the search | Examiner |
|---|---|---|
| The Hague | 4 July 2022 | Chehade, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 15 4762

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2022

| Patent document cited in search report | | Publication date | Patent family member(s) | | | Publication date |
|---|---|---|---|---|---|---|
| US 2010312144 | A1 | 09-12-2010 | NONE | | | |
| US 2016278670 | A1 | 29-09-2016 | EP | 3054844 | A1 | 17-08-2016 |
| | | | US | 2016278670 | A1 | 29-09-2016 |
| | | | WO | 2015052683 | A1 | 16-04-2015 |
| US 2021034841 | A1 | 04-02-2021 | NONE | | | |